# FASCICULE DE BREVET EUROPEEN

(11) **EP 1 749 082 B1**
(45) Date de publication et mention de la délivrance du brevet: **17.12.2014**
(21) Numéro de dépôt: 05773069.9
(22) Date de dépôt: 24.05.2005
(51) Int. Cl.: A61L 2/18, C11D 3/48, C11D 7/10

(54) **UTILISATION DU CUIVRE ET SES DÉRIVÉS POUR LA DECONTAMINATION DES PRIONS**
VERWENDUNG VON KUPFER UND SEINE DERIVATE ZUR DEKONTAMINATION VON PRIONEN
USE OF COPPER AND ITS DERIVATIVES FOR THE DECONTAMINATION OF PRIONS

(30) Priorité: 24.05.2004 FR 0405581
(43) Date de publication de la demande: 07.02.2007
(73) Titulaire: Centre National de la Recherche Scientifique (CNRS), 75794 Paris Cedex 16 (FR)
(72) Inventeur: LEHMANN, Sylvain, F-34170 CASTELNAU-LE-LEZ (FR); SOLASSOL, Jérôme, F-34070 MONTPELLIER (FR)
(74) Mandataire: Peaucelle, Chantal
(86) Numéro de dépôt international: PCT/FR2005/001283
(87) Numéro de publication internationale: WO 2005/118762

(56) Documents cités:
- WO-A-03/092745
- DE-C- 4 441 483
- US-A1- 2004 005 304

## Description

L'invention a pour objet des produits et procédés pour la décontamination de produits et matériaux infectés par des agents transmissibles non conventionnels (ATNC) responsables des encéphalopathies subaiguës spongiformes transmissibles.

L'invention vise plus spécialement la décontamination de produits et matériaux infectés par les prions qui s'accumulent principalement dans le cerveau de l'hôte, plus particulièrement par une isoforme anormale, PrP^{sc} (pour PrP^{scrapie}) qui résulte d'une modification conformationnelle d'une protéine PrP^{c} (PrP cellulaire) codée par l'hôte.

L'apparition d'un nouveau variant de la maladie de Creutzfeldt Jakob en Grande- Bretagne et son lien possible avec l'encéphalopathie spongiforme bovine ont mis en avant une possible contagiosité de la maladie, en particulier par des produits d'origine animale infectés, notamment par des aliments, et par des matériaux médico-chirurgicaux contaminés.

WO 94/04167 décrit une solution comprenant du cuivre pour inactiver des champignons, des bactéries et des virus, ne décrit pas de méthode pour décontaminer des produits et matériaux infectés par des prions.

En effet, les techniques actuelles de désinfection et de stérilisation ne permettent pas de détruire aisément les prions qui sont résistants à la plupart des procédés habituellement utilisés. Des Directives administratives ont édicté les règles à respecter en la matière, mais sont très lourdes à mettre en oeuvre, onéreuses et nécessitent l'utilisation de produits toxiques.

Au vu de ce contexte, les inventeurs ont cherché des composés permettant une décontamination de l'agent pathogène simple, efficace et utilisable sur une grande majorité de surfaces et matériaux médico-chirurgicaux. Leurs travaux ont montré l'efficacité de certains dérivés métalliques à cet égard.

L'invention a donc pour but l'utilisation de tels composés pour la décontamination de prions.

Elle vise également un procédé de traitement de matériaux et de produits infectés comprenant l'utilisation de ces composés.

L'invention vise ainsi l'utilisation du Cu et de ses dérivés, pour la décontamination de produits et matériaux infectés par de prions.

Selon une disposition supplémentaire de l'invention, ces dérivés sont mis en oeuvre avec H₂O₂.

De manière préférée, le dérivé métallique est CuSO₄.

Le ou les composés utilisés selon l'invention se présentent avantageusement sous forme de solutions aqueuses.

L'étude de l'action de ces dérivés sur la décontamination des agents pathogènes responsables des maladies à prions a montré leur grande efficacité pour décontaminer les produits ou matériaux infectés.

De manière avantageuse, il s'agit de surcroît de composés non toxiques, biodégradables aisément manipulables.

L'invention vise donc également un procédé pour la décontamination de produits ou de matériaux infectés par des prions, caractérisé en ce qu'il comprend leur mise en contact avec au moins un composé tel que défini ci-dessus, notamment le cuivre ou ses dérivés, ou d'une solution le renfermant, et le cas échéant avec H₂O₂.

Dans un mode préféré de réalisation de l'invention, le dérivé métallique est CuSO₄.

Le traitement de décontamination est réalisé de préférence avec une solution renfermant ledit composé, notamment ledit Cu ou ses dérivés, à raison d'au moins 500µM, notamment de 500 à 1000 µM.

H₂O₂, lorsqu'il est utilisé est présent dans les solutions à raison d'environ 50 mM.

Des résultats satisfaisants sont obtenus en opérant à température ambiante, pendant une durée de l'ordre de 15 à 60 min, notamment pendant environ 30 min.

Ce ou ces composés et leurs solutions présentent ainsi un grand intérêt dans le cadre d'un usage hospitalier, où ils permettent, notamment, une décontamination des dispositifs médicaux ou médicaux-chirurgicaux à risques, comme les matériaux à usages multiples, tels que les endoscopes, sondes (dialyse), ou encore une décontamination des surfaces de travail, type paillasse ou sol, également à risques.

Lesdits composés ou leurs solutions sont aussi particulièrement utiles pour décontaminer par exemple un matériel infectieux d'origine cérébrale et les produits biologiques provenant de sujets porteurs des formes infectieuses de la maladie de Creutzfeldt Jakob. L'invention vise également l'utilisation desdits composés et de leurs solutions pour décontaminer les produits d'origine sanguine ou du matériel biologique utilisé dans les greffes.

De manière avantageuse, ces composés et solutions se sont avérés efficaces sur la souche de prion liée à l'ESB (encéphalopathie spongiforme transmissible bovine ou maladie de la vache folle). Ils sont donc applicables à tout type de prion quelque soit la souche et l'origine de l'agent.

Ils sont également utilisables avec avantage dans le cadre d'une application agro-alimentaire, notamment pour la décontamination de tout composé potentiellement infectieux et en particulier des farines animales ou autres produits d'origine animale contaminés. Des applications d'intérêt comprennent également la décontamination de locaux, comme les abattoirs, de surface et appareils risquant d'être en contact avec les agents infectieux. On citera par exemple en particulier ceux provenant de ruminants.

D'autres caractéristiques et avantages de l'invention sont donnés dans les exemples qui suivent.

### Exemple 1 Solution de décontamination de prions renfermant CuSO₄ et H₂O₂

### a) Etude de l'action du cuivre associé à H₂O₂ sur la dégradation de la PrP^{Sc} présente dans des homogénats de cerveaux infectieux de souris.

Des solutions de CuSO₄ et de H₂O₂ à différentes concentrations sont ajoutées à des échantillons constitués par des extraits infectieux d'homogénats de cerveau murins et sont laissées en contact environ 30 min à température ambiante.

Les échantillons sont ensuite déposés sur un gel de polyacrylamide SDS-PAGE après ajustement des concentrations en protéines et digestion avec de la protéinase K (PK) à une concentration de 1 mg de PK pour 50 mg de protéine. La présence de PrP^{Sc} est révélée par Western blot.

On observe qu'à une concentration de 100µM de CuSO₄et de 50mM de H₂O₂ les échantillons infectieux présentent des taux diminués de PrP^{Sc}. A une concentration de 500µM de CuSO₄et de 50mM de H₂O₂, le taux de PrP^{Sc} présent dans les homogénats infectieux devient indétectable en Western blot. A cette concentration, l'effet est potentialisé par l'action de H₂O₂.

En utilisant des doses plus élevées de CuSO₄ de 1 mM à 10mM, le cuivre seul permet de faire disparaître le signal de PrP^{Sc} et H₂O₂ n' est plus nécessaire.

Ces résultats sont confirmés en répétant ces expériences sur des homogénats provenant de cerveaux de souris infectés par la souche de prion 22L.Des résultats similaires ont été obtenus sur des homogénats provenant de la souche murine Chandler et d'ESB, ce qui démontre que l'effet décontaminant n'est pas souche-dépendant.

### b) Etude de l'infectivité d'homogénats de cerveaux infectieux traités avec une forte concentration de cuivre in vitro

Des homogénats de cerveaux 22L ont été traités pendant environ 30 min avec différentes concentrations de CuSO₄ associé ou non à H₂O₂ à différentes concentrations.

Ces homogénats infectieux sont dialysés, puis déposés sur des cellules de neuroblastomes murins ayant la capacité de répliquer l'agent infectieux.

Des homogénats de cerveau non traité sont utilisés comme contrôle.

Un Western blot est réalisé après 6 passages sur les lysats cellulaires et après digestion avec de la PK, afin de tester la présence de PrP^{Sc} signifiant que les échantillons testés demeurent toujours infectieux.

Dans les résultats obtenus, on constate que l'infection est diminuée par le traitement.

### Exemple 2 Tests in vivo

Une confirmation des résultats observés *in vitro* a été réalisée. Des homogénats de cerveau infectieux 22L ont été traités notamment par 500 µM de CUSO₄ et 100 mM d'H₂O₂. Les homogénats, traités ou non, ont été inoculés à des souris par voie intracérébrale. Les animaux témoins inoculés avec les homogénats non traités sont tous tombés malades en 168 jours +/- 2 jours. Certains animaux inoculés avec les homogénats traités sont toujours vivants après plus de 300 jours. Ce résultat démontre qu'une réduction d'au moins 7 log du titre infectieux peut être obtenue par cette méthode de décontamination.

### Exemple 3 Décontamination de matériaux chirurgicaux

Des matériaux tels que des endoscopes sont immergés dans une solution renfermant 1mg de CuSO₄ pendant 20 min. Les tests effectués pour identifier la présence de prions après ce traitement se sont révélés négatifs.

### Exemple 4 Etude de l'efficacité de décontamination

On rapporte ci-après les données concernant l'efficacité de décontamination testée *in vivo* après inoculation à la souris. Pour cela des Homogénats de cerveaux contrôle et décontaminé ont été inoculés de façon intracérébrale à la souris (C57B1). Les temps de survie des souris sont les suivants :
1) Inoculation des homogénats infectieux contrôles : 167,6 +/-0,5 jours.
2) Homogénats traités au CuSO₄ (0,5mM, 30 min, température ambiante) : 200,2 +/- 8,9 jours.
3) Homogénats traités au CuSO₄ (1mM, 30 min, température ambiante) : 217,2 +/- 13,4 jours.
4) Homogénats traités au C_{U}SO₄ (1mM) + H₂O₂ (100mM, 30 min, température ambiante) : 266,4 +/- 15,6 jours.
Compte-tenu des ces durées d'incubation dans ces différentes conditions et d'une courbe de titration de l'agent infectieux réalisée sur ces souris, on peut estimer que la décontamination obtenue est supérieure à 10⁴ dans les homogénats traités juste par le CuSO₄ et supérieure à 10⁵ dans l'homogénat traité au CuSO₄ + H₂O₂.

## Revendications

1. Utilisation du Cu et de ses dérivés, pour la décontamination de produits et matériaux infectés par des prions.

2. Utilisation selon la revendication 1, **caractérisée en ce que** le cuivre ou ses dérivés sont mis en oeuvre avec H₂O₂.

3. Utilisation selon la revendication 1 ou 2, **caractérisée en ce que** le dérivé est CuSO₄.

4. Utilisation selon l'une quelconque des revendications 1 à 3, **caractérisée en ce que** le ou les dérivés utilisés se présentent sous forme de solutions aqueuses.

5. Procédé pour la décontamination de produits ou de matériaux infectés par des prions, **caractérisé en ce qu'**il comprend leur mise en contact avec le cuivre ou ses dérivés, ou une solution le renfermant, et le cas échéant avec en outre H₂O₂.

6. Procédé selon la revendication 5, **caractérisé en ce que** le dérivé est CuSO₄.

7. Procédé selon la revendication 5 ou 6, **caractérisé en ce que** le traitement de décontamination est réalisé avec une solution renfermant ledit Cu ou ses dérivés à raison d'au moins 500 µM, notamment de 500 à 1000 µM.

8. Procédé selon l'une quelconque des revendications 5 à 7, **caractérisé en ce que** H₂O₂ est présent dans les solutions à raison de 50 mM.

9. Utilisation selon l'une quelconque des revendications 1 à 4, pour la décontamination des dispositifs médicaux ou médicaux-chirurgicaux à risques, comme les matériaux à usages multiples, tels que les endoscopes, ou encore pour la décontamination des surfaces de travail, type paillasse ou sol.

10. Utilisation selon l'une quelconque des revendications 1 à 4, pour la décontamination de tout produit potentiellement infectieux et en particulier des farines animales ou autres produits d'origine animale contaminés.

## Patentansprüche

1. Verwendung von Cu und seinen Derivaten für die Dekontamination von mit Prionen infizierten Produkten und Materialien.

2. Verwendung nach Anspruch 1, **dadurch gekennzeichnet, dass** das Kupfer oder seine Derivate mit H₂O₂ eingesetzt werden.

3. Verwendung nach Anspruch 1 oder 2, **dadurch gekennzeichnet, dass** das Derivat CuSO₄ ist.

4. Verwendung nach einem der Ansprüche 1 bis 3, **dadurch gekennzeichnet, dass** das oder die verwendeten Derivate in Form von wässrigen Lösungen vorliegen.

5. Verfahren zur Dekontamination von mit Prionen infizierten Produkten oder Materialien, **dadurch gekennzeichnet, dass** es deren Inkontaktbringen mit dem Kupfer oder seinen Derivaten, oder einer es enthaltenden Lösung, sowie gegebenenfalls ferner mit H₂O₂ umfasst.

6. Verfahren nach Anspruch 5, **dadurch gekennzeichnet, dass** das Derivat CuSo₄ ist.

7. Verfahren nach Anspruch 5 oder 6, **dadurch gekennzeichnet, dass** die Dekontaminationsbehandlung mit einer Lösung, die das Kupfer oder seine Derivate in einer Menge von wenigstens 500 µM, insbesondere von 500 bis 1000 µM enthält, vollzogen wird.

8. Verfahren nach einem der Ansprüche 5 bis 7, **dadurch gekennzeichnet, dass** H₂O₂ in den Lösungen in einer Menge von 50 mM vorhanden ist.

9. Verwendung nach einem der Ansprüche 1 bis 4, für die Dekontamination von gefährdeten medizinischen oder medizinisch-chirurgischen Vorrichtungen, wie Mehrwegmaterialien, wie Endoskopen, oder aber für die Dekontamination von Arbeitsflächen, vom Typ Labortisch oder Boden.

10. Verwendung nach einem der Ansprüche 1 bis 4, für die Dekontamination von jedwedem potentiell infektiösen Produkt und insbesondere von Tiermehlen oder anderen kontaminierten Produkten tierischen Ursprungs.

## Claims

1. Use of Cu and of its derivatives, for the decontamination of products and materials infected by prions.

2. Use according to claim 1, **characterized in that** the copper and its derivatives are used with H₂O₂.

3. Use according to claim 1 or 2, **characterized in that** the derivative is CuSO₄.

4. Use according to any one of claims 1 to 3, **characterized in that** the derivative(s) used are in the form of aqueous solutions.

5. Method for the decontamination of products or materials infected by prions, **characterized in that** it comprises placing them in contact with the copper or its derivatives, or a solution containing it, and if necessary also with H₂O₂.

6. Method according to claim 5, **characterized in that** the derivative is CuSO₄.

7. Method according to claim 5 or 6, **characterized in that** the decontamination treatment is carried out with a solution containing said copper or its derivatives at a level of at least 500 µM, in particular from 500 to 1000 µM.

8. Method as claimed in any one of claims 5 to 7, **characterized in that** H₂O₂ is present in the solutions at a level of about 50 mM.

9. Use according to any one of claims 1 to 4, for the decontamination of risk medical or medical-surgical devices, such as multiple use materials, such as endoscopes, or also for the decontamination of work surfaces, such as a draining board or floor.

10. Use according to any one of claims 1 to 4, for the decontamination of any potentially infectious product and in particular animal meal or other contaminated products of animal origin.
